# EUROPEAN PATENT APPLICATION

(11) **EP 2 269 968 A1**
(43) Date of publication of application: **05.01.2011**
(21) Application number: 08873451.2
(22) Date of filing: 09.06.2008
(51) Int. Cl.: C07C 11/18, C07C 2/86

(54) **METHOD FOR REPROCESSING BY-PRODUCTS OF THE LIQUID PHASE SYNTHESIS OF ISOPRENE FROM ISOBUTHYLENE AND FORMALDEHYDE**

(30) Priority: 17.03.2008 RU 2008110238
(71) Applicant: Obshestvo S Ogranichennoi Otvetstvennoctiu "Eurochim-SPB-Trading", St.Petersburg 198095 (RU)
(72) Inventor: BUSYGIN, Vladimir Mikhailovich, Nizhnekamsk 423550 (RU); DYKMAN, Arkady Samuilovich, St.Petersburg 194356 (RU); POLYAKOV, Sergey Aleksandrovich, St.Petersburg 192177 (RU); GILMANOV, Khamit Khamisovich, Nizhnekamsk 423570 (RU)
(74) Representative: Henrion, Oliver
(86) International application number: PCT/RU2008/000368
(87) International publication number: WO 2009/116890

(57) **Abstract**

The invention is directed to the increasing of selectiveness of the process (UYUP), increasing of conversion of raw materials and heavy residue, and also the depth of processing.

The indicated technical result is achieved by that way, that the method of processing of co-products of liquid-phase synthesis of isoprene from formaldehyde or formaldehyde-containing products is carried out at the temperature of 400-480°C in the presence of the water vapor with the aluminum silicate-containing catalyst with the further heating up to the temperature of 400-550°C, in this case, the process is carried out at the initial temperature, that is 5-40°C lower and the final temperature, that is 5-40°C higher, than the average temperature of contacting during the progressive increasing of the temperature from the initial au to the final one and during the constant reducing of the feed space velocity at the beginning of contacting 3-15 % higher, and at the end of contacting 3-15 % lower, than the middling cycling feed space velocity (table 4).

## Description

The present invention relates to the area of oil and gas technology, exactly to the method of obtaining of isoprene, isobutylene and formaldehyde of co-products of production of isoprene. It may find application in the industry of artificial rubber and in organic synthesis.

Until recently, the two-stage process of production of isoprene from isobutylene and formaldehyde had wide-spread occurrence. At the first stage, during interaction of isobutylene with formaldehyde in the presence of acid catalyst, 4,4-dimethyl-dioxane-1,3 (DMD) and co-products, representing, mainly, dioxane alcohols and their derivatives, appear. The indicated co-products boil at higher temperatures, than DMD, and, therefore, got the name of higher boiler co-products of synthesis of isoprene (HCP).

At the second stage of the process, DMD is dissolved in isoprene into calcium-boron-phosphate-containing catalysts in the presence of water vapor at 250 - 450°C. In this case, formaldehyde, isobutylene, iso-propenyl-ethyl alcohol (IPEA), methyl-dehydro-pyran (MDHP), methylene tetrahydropyran (MTHP), green oil and the other substances appear as co-products. Formaldehyde, isobutylene and IPEA are delivered to the recycle, green oil is burnt, and the fraction of MDHP, representing the mixture of CMeCb MDHP, MTHP and light components with the boiling temperature of 40-85°C (so-called hexadienic fraction) is delivered to the catalytic dissolution. The yield of HCP composes 400-450 kg for 1 t of isoprene. The part of HCP finds qualified application (for example, as flotation agent), the light part of HCP is dissolved in isoprene and raw products of synthesis, and the remaining part is burnt.

The method of processing of co-products of synthesis of isoprene by catalytical splitting of the fraction of HCP (T_{boil.} 150-300°C) at the constant temperature of 400°C is known. The oxide of silicon and aluminum silicate are used as the catalyst (patent of Japan Nº 49-38249, published on 16.10.1974). The yield of isoprene achieves 14-17 %, yield of formaldehyde - 27-33 %.

The disadvantage of the method - significant carbon deposit, sophistication of the technology at the expense of the long oxidation catalyst regeneration and low yield of the target products.

The method of processing of HCP is known by joint dissolution of HCP and 5-70 % of the fraction of MDHP, separating from the catalysate, receiving during vaporphase heterogeneous dissolution of DMD into calcium-boron-phosphate-containing catalyst, from which the fraction is previously separated, boiling up to the temperature of 40-85°C, successively above two catalysts - hard contact with the specific surface area of 0,2-1,0 m²/g and oxide aluminum silicate-containing catalyst C-84 of the following composition, % mas.:

| | | |
|---|---|---|
| Al₂O₃ | - | 5,0 - 30,0 |
| Fe₂O₃ | - | 0,1 - 5,0 |
| MgO | - | 0,1 - 5,0 |
| CaO | - | 0,1 - 5,0 |
| K₂O | - | 0,1 - 3,0 |
| Na₂O | - | 0,1 - 3,0 |
| TiO₂ | - | 0,1 - 3,0 |
| SiO₂ | - | the rest, |

taken in the proportion (0,05 - 0,3) : 1, respectively.

The process in carried out in the interval of temperatures of 200 - 480°C in the presence of water vapor at the constant temperature of contacting and constant feed space velocity (patent of RF Nº 1695631, published in 1996). The light part of the fraction of HCP (light fraction of HCP) and fraction of MDHP, received from the catalysate, appearing during heterogeneous dissolution of DMD, from which the fraction is previously separated, boiling up to the temperature of 40 - 85°C, are used as raw materials.

In the indicated method, the conversion degree of HCP, productivity of the process, duration of the cycle of contacting are increased, however, the increased carbon deposit at the level of 2,0 % mas., and also small conversion of the heavy residue (∼80 %) are mentioned, that leads to the plugging of the system of condensation and low ultimate yield of useful products (UYUP) (isobutylene, isoprene, formaldehyde, 2-methylpropan-2-ol, 3-methyl-1-buten-3-ol, 3-methyl-3-buten-1-ol, 4-methylene-tetrahydro-a-pyran, 4-methyl-5,6-dehydro-a-pyran, 4,4-dimethyl-dioxane-1,3, 3-methyl-butandiol-1,3) - 81,0-81,5%.

The method of processing of co-products, carrying out in the interval of temperatures of 350-550°C, allows to increase UYUP and reduce carbon deposit, in the presence of water vapor and 0,2-5,0 % of ammonia at the catalytical composition, consisting of the hard contact with the specific surface area of 0,2-1,0 m²/g and aluminum silicate-containing catalyst C-84 of the following composition, % mas.:

| | | |
|---|---|---|
| Al₂O₃ | - | 5,0 - 30,0 |
| Fe₂O₃ | - | 0,1 - 5,0 |
| MgO | - | 0,1 - 5,0 |
| CaO | - | 0,1 - 5,0 |
| K₂O | - | 0,1 - 3,0 |
| Na₂O | - | 0,1 - 3,0 |
| TiO₂ | - | 0,1 - 3,0 |
| SiO₂ | - | the rest. |

In this case, the catalytical composition consists of four layers of the mentioned above components.

The process is carried out at the constant temperature of contacting and feed space velocity.

HCP, or technical fraction of MDHP, received from the catalysate, appearing during the heterogeneous-catalytical dissolution of DMD, or their mixture, are used as raw co-products (patent of RF Nº 2134679, published in 1999).

The disadvantage of the method is also the increased carbon deposit - 1,8%, low selectiveness of the process and small conversion of the heavy residue (∼78-80%).

The method of processing of co-products of synthesis of isoprene from isobutylene and formaldehyde - HCP and/or pyran fraction by splitting of raw products in the interval of temperatures of 350-450°C is also known, in the presence of water vapor with the aluminum silicate-containing catalyst «C-97», containing, % mas.:

| | | |
|---|---|---|
| Al₂O₃ | - | 5,0 - 30,0 |
| Fe₂O₃ | - | 0,4 - 5,0 |
| MgO | - | 0,4 - 5,0 |
| CaO | - | 5,2 - 7,0 |
| K₂O | - | 1,0 - 3,0 |
| Na₂O | - | 1,0 - 3,0 |
| TiO₂ | - | 0,4 - 1,0 |
| SiO₂ | - | the rest, |

or with the catalyst of the above mentioned composition jointly with the hard contact - nonporous material with the special surface area of 0,2-1,0 m²/g with the proportion - hard contact : catalyst - (0,05-0,3) :1. (Patent of RF Nº 2167710, published on 27.05.2001).

The process is carried out at the constant temperature of contacting and feed space velocity.

For splitting, the general fraction of HCP is used, obtaining at the stage of synthesis of DMD with recirculation of the water layer, or the light fraction of HCP, separating from the general fraction of HCP and containing, mainly, dioxane alcohols, or pyran fraction, from which the fraction of hexadienics with the boiling temperature of 40-85°C, or the mixture of HCP and pyran fraction is previously separated.

To the number of disadvantages of the present method of processing of co-products, shallow depth of conversion of the heavy residue - 75%, increased carbon deposit and low UYUP should be also related.

The closest analogue is the method of processing of co-products of production of isoprene - fraction of MDHP, separating from the catalysate of the process of dissolution of DMD into calcium- boron-phosphate-containing catalysts with the preliminary stripping from it the products with the boiling temperature up to 80°C, or fraction of HCP, received at the first stage of synthesis of isoprene from isobutylene and formaldehyde with recirculation of the water layer, or MDHP in the mixture with HCP, with the aluminum silicate-containing catalysts «C-84» or «C-97» (composition of which is shown above) in the interval of temperatures of 400-480°C in the presence of water vapor at the constant temperature of contacting and feed space velocity with preliminary dilution of raw materials and their heating before supplying in the zone of contacting up to the temperature of 400-550°C (patent of RF Nº 2278105).

To the number of disadvantages of the present invention, the increased carbon deposit, small amount of conversion of raw materials and heavy residue, and also shallow depth of conversion of unidentified products - «x»-es and, as the result of this, low UYUP, should be related.

With the purpose of the further selectivity improving of the process (UYUP), increasing of conversion of raw materials and heavy residue, and also depth of processing of «x»-es, it is offered to carry out the processing of co-products, appearing in the process of liquid-phase synthesis of isoprene from isobutylene and formaldehyde and formaldehyde-containing products, in particular, DMD, in the interval of temperatures of 400-480°C in the presence of water vapor with the aluminum silicate-containing catalyst C-84 or C-97 with the further heating up to the temperature of 400-550°C during conducting of the process 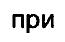 at the initial temperature, which is 5-40°C lower and the final temperature, which is 5-40°C higher, than the average temperature of contacting during the progressive increasing of the temperature from initial up to the final and during the constant reducing of the speed space velocity at the beginning of contacting for 3-15 % higher, and at the end of contacting for 3-15 % lower, than the middling-cycling speed space velocity.

While using of the fraction of MDHP or its mixture with HCP as co-products, the fraction of MDHP is previously evaporated in the evaporator in the presence of the water vapor, and HCP is added in the appearing gas-vapor mixture.

The fraction of MDHP, appearing in the liquid-phase process of obtaining of isoprene from isobutylene and formaldehyde by interaction of formaldehyde or formaldehyde-containing products, in particular, DMD and TMC and (or) isobutylene, at 140-170°C in the presence of acid catalyst, or the fraction of HCP, appearing during synthesis of DMD, or their mixture, are used as co-products.

As the fraction of HCP, the general fraction of HCP, or its light part, obtained by distillation and consisting, mainly, of dioxane alcohols, may be used.

The distinguishing features of the offered method are conducting of the process at the initial temperature of contacting, which is 5-40°C lower and the final temperature, which is 5-40°C higher, than the average temperature of contacting during the progressive increasing of the temperature from initial up to the final and during the constant reducing of the speed space velocity at the beginning of contacting for 3-15 % higher, and at the end of contacting for 3-15 % lower, than the middling-cycling speed space velocity.

The industrial application of the offered method is confirmed by the following examples.

### Example Nº 1.

As the initial co-product, the fraction of MDHP is used, obtained during interaction of DMD and TMC at 165°C in the presence of 6% of phosphoric acid (the composition of the fraction is shown in the table 1).

### Table 1.

**Composition of the fraction of MDHP**

| Name of components | % mas. |
|---|---|
| TMC | 0,5 |
| DMVK | 6,8 |
| dimers | 0,3 |
| IBC | 1,4 |
| MTHP | 2,6 |
| MDHP | 51,4 |
| XX before DMD | 28,3 |
| DMD | 3,8 |
| MBD | 0,0 |
| Pyran alcohol | 0,0 |
| Dioxane alcohol 1 | 0,0 |
| Dioxane alcohol 2 | 0,0 |
| Dioxane alcohol 3 | 0,0 |
| XX after DMD | 4,3 |
| Heavy residue | 0,6 |
| Sum | 100,0 |

The indicated products are diluted by the water vapor at the proportion - fraction of MDHP : H₂O ∼ 1 : 0,3 (% mas.) and are heat up to 400°C with adding of the water vapor to the flow, until receiving of the proportion - fraction of MDHP: H₂O = 1 : 2,0 (mas.), after that are delivered to the reactor with the catalyst C-97, loaded in it, containing, % mas.

| | | |
|---|---|---|
| Al₂O₃ | - | 22,0 |
| Fe₂O₃ | - | 0,4 |
| MgO | - | 1,0 |
| CaO | - | 5,7 |
| K₂O | - | 1,0 |
| Na₂O | - | 3,0 |
| TiO₂ | - | 1,0 |
| SiO₂ | - | the rest. |

The process of processing of MDHP is carried out at the beginning of the cycle at the temperature of 395°C and at the end of the cycle at the temperature of 405°C and the initial feed speed velocity of 1,03 hour⁻¹ and the final feed speed velocity at the end of the cycle of contacting of 0,97 hour⁻¹. In this case, in the course of the cycle of contacting, the temperature is progressively increased, and the space velocity is reduced. The duration of the cycle of contacting composes 3 hours. The proportion - raw materials : water - composes 1,0 : 3,0. After the cycle of contacting, the catalyst is regenerated by the gas-vapor mixture at 500°C.

The results of the experience are shown in the table 4, experience 1.

### Example Nº 2.

The process of processing of co-products is carried out in the same way, as in the example Nº 1, except, that the initial temperature of the process composes 360°C, and the final one - 440°C, in this case, the feed space velocity at the beginning of the cycle of contacting composes 1,15 hour⁻¹, and at the end of the cycle of contacting - 0,85 hour⁻¹. The catalyst C-84 is used in the process of processing of co-products.

The results of the experience are shown in the table 4, experience 2.

### Example Nº 3.

The process of processing of co-products is carried out in the same way, as in the example Nº 1, except, that the initial temperature of the process composes 410°C, and the final one - 490°C, in this case, the feed space velocity at the beginning of the cycle of contacting composes 1,15 hour⁻¹, and at the end of the cycle of contacting - 0,85 hour⁻¹. As raw material, the pyran fraction is used, the composition of which is shown in the table 2. The catalyst C-84 is used in the process of processing of co-products.

The results of the experience are shown in the table 4, experience 3.

### Example Nº 4.

The process of processing of co-products is carried out in the same way, as in the example Nº 1, except, that the temperature of the process is constant and composes 450°C, in this case, the feed space velocity composes 1,0 hour⁻¹. As raw material, the pyran fraction is used, the composition of which is shown in the table 2. The catalyst C-84 is used in the process of processing of co-products.

The results of the experience are shown in the table 4, experience 4.

### Example Nº 5.

As raw material, the fraction of HCP is used, the composition of which is shown in the table 3. The process of processing of co-products is carried out in the same way, as in the example Nº 1, except, that the initial temperature of the process composes 410°C, and the final one - 490°C, in this case, the feed space velocity at the beginning of the cycle of contacting composes 1,15 hour⁻¹, and at the end of the cycle of contacting - 0,85 hour⁻¹. The catalyst C-84 is used in the process of processing of co-products.

The results of the experience are shown in the table 4, experience 5.

### Example Nº 6.

75% mas. of HCP (composition is shown in the table 3) are injected to the evaporated vapor-raw mixture, containing the fraction of MDHP (composition is shown in the table 1), and composing 25 % from HCP, supplied to it, in this case, the proportion - raw materials (HCP + MDHP) : H₂O (mas.) before the overheating composes 1,0 : 2,0. The indicated vapor-raw mixture is heat up to the temperature of 500°C, after that it is supplied to the reactor, loaded by the catalyst C-97.

The process is carried out in the same way, as in the example Nº 1, except, that the initial temperature of contacting composes 410°C, and the final one - 490°C, the feed space velocity at the beginning of the cycle of contacting composes - 1,15 hour⁻¹, and at the end of the cycle of contacting - 0,85 hour⁻¹.

The results of the experience are shown in the table 4, (experience 6).

The ultimate yield of useful products during processing of the pyran fraction with reduced content of MDHP composed 70,1-70,8 % mas.(examples 1,2) while using of raw materials (table 1) with reduced content of MDHP.

During the processing of the fraction of HCP (example 5), the conversion of raw materials composed 96,4% mas., of the heavy residue - 88,6 % mas., of UYUP - 87,9 % mas.

During the joint processing of the pyran fraction (25%) and the fraction of HCP (75%) (example 6), the conversion of raw materials composed 97,1 % mas., of the heavy residue - 88,4% mas., of UYUP - 86,1 % mas.

While using of the pyran fraction (table 2) with the higher content of MDHP (example 3), the ultimate yield of useful products composed 86,7 % mas., that is 3,1 % mas. higher, than during the processing of raw materials at the constant temperature and feed speed velocity (example 4), in this case, the conversion of raw materials increased for 0,3 % mas.

Thus, the application of the mentioned above method of the process of processing of co-products of synthesis of isoprene allows to increase ultimate yield of useful products during the processing of the pyran fraction, fraction of HCP and their mixture.

## Claims

1. A method of processing by-products of liquid-phase synthesis of isoprene from isobutylene and formaldehyde or formaldehyde-containing products, in particular 4,4-dimethyl-1,3-dioxane, in a temperature interval of 400-480°C in the presence of water vapor on an aluminum silicate-containing catalyst with preliminary heating of the by-products to a temperature of 400-550°C in the presence of water vapor, **characterized in that** the process is carried out at an initial temperature, that is 5-40°C lower and a final temperature, that is 5-40°C higher, than the average contacting temperature, with a gradual increasing of the temperature of from the initial to the final temperature, and with continous reducing of the space velocity of feeding the starting material being at the beginning of the contacting cycle 3-15 % higher and at the end of the cycle 3 - 15 % lower, than the average cycle space velocity of feeding the starting material.

2. The method of claim 1, **characterized in that**, when using as the by-products a fraction of 4-methyl-5,6-dehydro-α-pyran being formed in a liquid-phase interaction of formaldehyde or formaldehyde-containing products, in particular 4,4-dimethyl-1,3-dioxane and trimethyl carbinol and/or isobutylene or a mixture of a fraction of 4-methyl-5,6-dihydro-α-pyran, with high boiling by-products, , the fraction of 4-methyl-5,6-dihydro-α-pyran is preliminary evaporated in an evaporator in the presence of water vapor.

3. The method of claim 1, **characterized in that**, industrial catalysts C-84 or C-97 are used as the aluminum silicate-containing catalyst.
